# EUROPÄISCHE PATENTSCHRIFT

(11) **EP 3 695 818 B1**
(45) Veröffentlichungstag und Bekanntmachung des Hinweises auf die Patenterteilung: **17.05.2023**
(21) Anmeldenummer: 20155997.8
(22) Anmeldetag: 07.02.2020
(51) Int. Cl.: A61F 9/008

(54) **COMPUTERPROGRAMM ZUR STEUERUNG EINES AUGENCHIRURGISCHEN LASERS UND BEHANDLUNGSVORRICHTUNG**
COMPUTERPROGRAM FOR CONTROLLING AN OPHTHALMIC SURGICAL LASER AND TREATMENT DEVICE
PROGRAMME D`ORDINATEUR DE COMMANDE D'UN LASER CHIRURGICAL OPHTALMIQUE ET DISPOSITIF D'ÉCLAIRAGE

(30) Priorität: 15.02.2019 DE 102019103851
(43) Veröffentlichungstag der Anmeldung: 19.08.2020
(73) Patentinhaber: Schwind Eye-Tech-Solutions GmbH, 63801 Kleinostheim (DE)
(72) Erfinder: ARBA MOSQUERA, Samuel, 63739 Aschaffenburg (DE); NAUBEREIT, Pascal, 63743 Aschaffenburg (DE); WENDLER, Thomas, 63811 Stockstadt (DE)
(74) Vertreter: Hofstetter, Schurack & Partner

(56) Entgegenhaltungen:
- EP-A1- 3 427 705
- DE-A1-102007 019 814
- DE-A1-102007 019 815
- US-B1- 6 325 792

## Beschreibung

Die vorliegende Erfindung betrifft ein Computerprogramm zur Steuerung eines augenchirurgischen Lasers für die Abtrennung eines Volumenkörpers mit vordefinierten Grenzflächen aus einer menschlichen oder tierischen Hornhaut. Die Erfindung betrifft weiterhin eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption und mindestens einer Steuereinrichtung für den oder die Laser sowie ein computerlesbares Medium.

Trübungen und Narben innerhalb der Hornhaut, die durch Entzündungen, Verletzungen oder angeborene Erkrankungen entstehen können, beeinträchtigen das Sehvermögen. In der DE 10 2007 019 815 A1 wird ein Verfahren zur Augenhornhaut-Transplantation beschrieben, um mittels der Transplantation Veränderungen der Augenhornhaut zu korrigieren. Die DE 10 2007 019 814 A1 beschreibt eine Planungseinrichtung für ein augenchirurgisches Verfahren zur refraktiven Korrektur, wobei voroperative Schnitte berücksichtigt werden. insbesondere für den Fall, dass diese krankhaften und/oder unnatürlich veränderten Bereiche der Hornhaut in der Sehachse des Auges liegen, wird eine klare Sicht erheblich gestört. In bekannter Art und Weise werden die so veränderten Bereiche durch eine sogenannte phototherapeutische Keratektomie (PTK) mittels eines ablativ wirkenden Lasers, zum Beispiel einem Excimer-Laser, beseitigt. Dies ist jedoch nur möglich, wenn die krankhaften und/oder unnatürlich veränderten Bereiche der Hornhaut in den oberflächlichen Schichten der Hornhaut liegen. Tieferliegende Bereiche, insbesondere innerhalb der Stroma, sind mittels ablativer Laserverfahren nicht erreichbar. Hier müssen zusätzliche Maßnahmen, wie zum Beispiel die Freilegung der tieferliegenden Bereiche mittels eines zusätzlichen Hornhautschnitts, ergriffen werden. Durch die zusätzlichen Maßnahmen wird nachteiligerweise die Behandlungsdauer deutlich erhöht. Zudem besteht die Gefahr, dass es durch die zusätzlichen Hornhautschnitte zu weiteren Komplikationen, wie zum Beispiel dem Auftreten von Entzündungen an den Schnittstellen kommt.

Es ist daher Aufgabe der vorliegenden Erfindung eine Steuerung eines augenchirurgischen Lasers für die Abtrennung eines Volumenkörpers mit vordefinierten Grenzflächen aus einer menschlichen oder tierischen Hornhaut bereitzustellen, mit denen die Nachteile des Standes der Technik überwunden werden.

Diese Aufgabe wird durch die unabhängigen Patentansprüche gelöst.

Vorteilhafte Ausgestaltungen mit zweckmäßigen Weiterbildungen der Erfindung sind in den jeweiligen Unteransprüchen angegeben, wobei vorteilhafte Ausgestaltungen des Computerprogramms als vorteilhafte Ausgestaltungen der Behandlungsvorrichtung, und des computerlesbaren Mediums und umgekehrt anzusehen sind.

Ein erster Aspekt der Erfindung betrifft ein Computerprogramm zum Definieren eines Musters anhand eines oder mehrerer Steuerdatensätze zur Steuerung eines augenchirurgischen Lasers für die Abtrennung eines Volumenkörpers mit vordefinierten Grenzflächen aus einer menschlichen oder tierischen Hornhaut, wobei der oder die Steuerdatensätze Steuerdaten zur Positionierung und/oder Fokussierung einzelner Laserpulse in der Hornhaut umfassen. Im Computerprogramm ist vorgesehen, dass die Grenzflächen des abzutrennenden Volumenkörpers durch das vordefinierte Muster definiert sind und die Grenzflächen mittels des Photodisruption erzeugt werden. Dabei werden die Grenzflächen des Volumenkörpers derart bestimmt, dass ein krankhafter und/oder unnatürlich veränderter Bereich innerhalb des Stroma der Hornhaut umschlossen wird. Durch das Muster wird mindestens ein Schnitt oder mindestens eine Öffnung in einem vordefinierten Winkel und mit einer vordefinierten Geometrie in der Hornhaut definiert, wobei der Schnitt oder die Öffnung eine Grenzfläche des Volumenkörpers schneidet und bis zu einer Oberfläche der Hornhaut ausgebildet ist, so dass der Volumenkörper über den Schnitt oder die Öffnung aus der Hornhaut entfernbar ist und wobei die Oberfläche der Hornhaut eine mittels Abtragen oder Verschieben einer obersten Hornhautschicht und/oder mittels Herstellung einer Hornhautklappe künstlich erzeugte Oberfläche des Auges ist. Damit ist das erfindungsgemäße Computerprogramm für eine Vielzahl von phototherapeutische Keratektomieverfahren verwendbar. Die Anwendungsmöglichkeiten werden deutlich erhöht.

Durch das erfindungsgemäße Computerprogramm ist es möglich, krankhafte und/oder unnatürlich veränderte Bereiche in der Stroma der Hornhaut, das heißt in tieferliegenden Bereichen der Hornhaut, zuverlässig zu entfernen. Eine zusätzliche Freilegung dieser tieferliegenden Bereiche der Hornhaut mittels zusätzlicher Hornhautschnitte ist grundsätzlich nicht notwendig. Dadurch kann die Behandlungsdauer deutlich verkürzt werden, mögliche Komplikationen durch die üblicherweise erforderlichen zusätzlichen Hornhautschnitte werden vermieden.

Der Volumenkörper kann dabei lentikelartig ausgebildet sein, wodurch ein einfaches Entfernen über den genannten Schnitt oder die genannte Öffnung möglich ist. Dadurch, dass der abzutrennende Volumenkörper lediglich durch die Grenzflächen beschrieben und definiert ist und diese Grenzflächen einerseits das krankhafte und/oder unnatürlich veränderte Gewebe beziehungsweise den entsprechend veränderten Bereich umschließen und andererseits mittels Photodisruption erzeugt werden, kann auf einen vollflächigen beziehungsweise vollvolumigen Abtrag des Volumenkörpers verzichtet werden. Es werden lediglich die Grenzflächen mittels Photodisruption erzeugt, sodass anschließend der vordefinierte Volumenkörper aus der Hornhaut entnommen werden kann. Unter dem Begriff "Grenzflächen" ist auch zu verstehen, dass der Volumenkörper gegebenenfalls mittels einer einzigen in der Hornhaut liegenden Grenzfläche definiert und abgetrennt werden kann. Durch das erfindungsgemäße Computerprogramm können einerseits phototherapeutische Keratektomie-Verfahren in tiefen Bereichen der Hornhaut, insbesondere der Stroma, durchgeführt werden. Andererseits wird die Behandlungsdauer für die Abtrennung des Volumenkörpers verkürzt, zudem wird auch der Energieeintrag in die Hornhaut des Patienten signifikant reduziert. Der lentikelartige Volumenkörper kann dabei in einem konzentrisch um ein Zentrum der Hornhaut angeordneten Bereich oder konzentrisch zu einer optischen Achse des Auges angeordnet sein. Es ist aber auch möglich, dass der lentikelartige Volumenkörper in einem nicht konzentrisch um das Zentrum der Hornhaut angeordneten Bereich oder nicht konzentrisch zu der optischen Achse des Auges angeordnet ist.

In weiteren vorteilhaften Ausgestaltungen des erfindungsgemäßen Computerprogramms wird der Laser derart gesteuert, dass das vordefinierte Muster zumindest teilweise kreis- und/oder spiralförmig abgetragen wird. Dabei kann der Start der Photodisruption durch die einzelnen Laserpulse im Zentrum der jeweiligen Grenzfläche oder auch am Rand der jeweiligen Grenzfläche erfolgen.

Erfindungsgemäß wird das vordefinierte Muster anhand eines oder mehrerer Steuerdatensätze definiert, wobei der oder die Steuerdatensätze Steuerdaten zur Positionierung und/oder zur Fokussierung einzelner Laserpulse in der Hornhaut umfassen. Die Ermittlung der Steuerdatensätze ist bekannt und ergeben sich insbesondere aus der topografischen und/oder pachymetrischen Vermessung der zu behandelnden Hornhaut sowie der Art, der Lage und des Umfangs des krankhaften und/oder unnatürlich veränderten Bereichs innerhalb der Stroma der Hornhaut. Insbesondere werden die Steuerdatensätze zumindest durch ein Bereitstellen von topographischen und/oder pachymetrischen und/oder morphologischen Daten der unbehandelten Hornhaut und ein Bereitstellen von topographischen und/oder pachymetrischen und/oder morphologischen Daten des zu entfernenden krankhaft und/oder unnatürlich veränderten Bereichs innerhalb der Hornhaut, erzeugt.

In weiteren vorteilhaften Ausgestaltungen des erfindungsgemäßen Computerprogramms ist der abzutrennende Volumenkörper derart ausgebildet, dass durch die Entfernung des Volumenkörpers zudem eine refraktive Korrektur des Auges erfolgt. Bei den genannten Fehlsichtigkeiten des Auges kann es sich um Myopie, Hyperopie, Presbyopie, Astigmatismus oder auch andere Fehlsichtigkeiten des Auges handeln. Weiterhin besteht die Möglichkeit, dass der krankhaft veränderte Bereich innerhalb der Hornhaut eine Trübung und/oder eine Narbe ist.

In weiteren vorteilhaften Ausgestaltungen des erfindungsgemäßen Computerprogramms ist die Steuereinrichtung derart ausgebildet, dass der Laser Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt. Derartige Laser werden bereits für photodisruptive Verfahren in der Augenchirurgie verwendet. So beschreibt beispielsweise die EP 2 211 803 B1 einen entsprechenden Femtosekundenlaser, der zur Herstellung eines so genannten Lentikels, das heißt eines linsenartigen Volumenkörpers, innerhalb der Hornhaut verwendet wird. Das so hergestellte Lentikel wird anschließend über einen Schnitt in der Hornhaut aus dieser entnommen. Die Verwendung derartiger photodisruptiver Laser anstelle von ablativ wirkenden Lasern bei der phototherapeutische Keratektomie (PTK) ist jedoch neu und aus dem Stand der Technik nicht bekannt. Die Verwendung von photodisruptiven Lasern bei dem erfindungsgemäßen Computerprogramm weist zudem den Vorteil auf, dass die Bestrahlung der Hornhaut nicht in einem Wellenlängenbereich unter 300 nm erfolgen muss. Dieser Bereich wird in der Lasertechnik unter dem Begriff "tiefes Ultraviolett" subsumiert. Dadurch wird vorteilhafterweise vermieden, dass durch diese sehr kurzwelligen und energiereichen Strahlen eine unbeabsichtigte Schädigung der Hornhaut erfolgt. Photodisruptive Laser der hier verwendeten Art bringen üblicherweise gepulste Laserstrahlung mit einer Pulsdauer zwischen 1 fs und 1 ns in das Hornhautgewebe ein. Dadurch kann die für den optischen Durchbruch notwendige Leistungsdichte des jeweiligen Laserpulses räumlich eng begrenzt werden, so dass eine hohe Schnittgenauigkeit bei der Erzeugung der Grenzflächen gewährleistet ist.

Ein zweiter Aspekt der vorliegenden Erfindung betrifft eine Behandlungsvorrichtung mit mindestens einem augenchirurgischen Laser für die Abtrennung eines vordefinierten Hornhautvolumens mit vordefinierten Grenzflächen eines menschlichen oder tierischen Auges mittels Photodisruption und mindestens einer Steuereinrichtung für den oder die Laser, die ausgebildet ist, die Schritte des Computerprogramms gemäß dem ersten Aspekt der Erfindung auszuführen. Die erfindungsgemäße Behandlungsvorrichtung ermöglicht es, dass die bei der Verwendung üblicher ablativer Behandlungsvorrichtungen auftretenden Nachteile, nämlich relativ lange Behandlungszeiten und ein relativ hoher Energieeintrag durch den Laser in die Hornhaut, zuverlässig vermieden werden. Diese Vorteile werden insbesondere durch die Ausbildung des augenchirurgischen Lasers als photodisruptiver Laser erzielt.

Dabei ist der Laser geeignet, Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

In weiteren vorteilhaften Ausgestaltungen der erfindungsgemäßen Behandlungsvorrichtung umfasst die Steuereinrichtung mindestens eine Speichereinrichtung zur zumindest temporären Speicherung von mindestens einem Steuerdatensatz, wobei der oder die Steuerdatensätze Steuerdaten zur Positionierung und/oder zur Fokussierung einzelner Laserpulse in der Hornhaut umfassen; und mindestens eine Strahleneinrichtung zur Strahlführung und/oder Strahlformung und/oder Strahlablenkung und/oder Strahlfokussierung eines Laserstrahls des Lasers aufweist. Die genannten Steuerdatensätze werden dabei üblicherweise anhand einer gemessenen Topografie und/oder Pachymetrie und/oder Morphologie der zu behandelnden Hornhaut und der Art des zu entfernenden, krankhaft und/oder unnatürlich veränderten Bereichs innerhalb der Hornhaut, erzeugt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Ein dritter Aspekt der Erfindung betrifft ein computerlesbares Medium, auf dem das Computerprogramm gemäß dem ersten Erfindungsaspekt gespeichert ist. Weitere Merkmale und deren Vorteile sind den Beschreibungen des ersten und zweiten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Zudem betrifft die Erfindung ein Computerprogramm zur Abtrennung eines Volumenkörpers mit vordefinierten Grenzflächen aus einer menschlichen oder tierischen Hornhaut, umfassend Befehle, die bewirken, dass eine Behandlungsvorrichtung den Laser mittels einer Steuereinrichtung derart steuert, dass dieser gepulste Laserpulse in einem vordefinierten Muster in die Hornhaut abgibt, wobei die Grenzflächen des abzutrennenden Volumenkörpers durch das vordefinierte Muster definiert sind und die Grenzflächen mittels Photodisruption erzeugt werden, wobei die Grenzflächen des Volumenkörpers derart bestimmt werden, dass ein krankhafter und/oder unnatürlich veränderter Bereich innerhalb der Stroma der Hornhaut umschlossen wird, wobei der Laser derart gesteuert wird, dass mindestens ein Schnitt in einem vordefinierten Winkel und mit einer vordefinierten Geometrie in der Hornhaut erzeugt wird, wobei der Schnitt eine Grenzfläche des Volumenkörpers schneidet und bis zu einer Oberfläche der Hornhaut ausgebildet ist, so dass der Volumenkörper über den Schnitt oder die Öffnung aus der Hornhaut entfernbar ist und wobei die Oberfläche der Hornhaut eine mittels Abtragen oder Verschieben einer obersten Hornhautschicht und/oder mittels Herstellung einer Hornhautklappe künstlich erzeugte Oberfläche des Auges ist.

Das erfindungsgemäße Computerprogramm ist für eine Vielzahl von Verfahren bei der Korrektur von Fehlsichtigkeiten des Auges verwendbar. Insbesondere bei der photorefraktiven Keratektomie (PRK), bei der Laser-epithelialen Keratomileusis (LASIK), der epithelialen Laser-in-situ-Keratomileusis (Epi-LASIK) oder der transepithelialen photorefraktiven Keratektomie (Trans-PRK) kann das erfindungsgemäße Computerprogramm verwendet werden. Es handelt sich dabei um ein Computerprogramm, bei dem ein Gewebeabtrag unter anderem unterhalb einer künstlich erzeugten Hornhautoberfläche stattfindet. Im Gegensatz zu dem bekannten Computerprogramm zur Gewebeabtragung der künstlichen Hornhautoberfläche wird bei dem erfindungsgemäßen Computerprogramm der Laser jedoch derart gesteuert, dass keine vollflächige Abtragung eines vordefinierten Volumenkörpers der Hornhaut erfolgt, sondern der Volumenkörper durch die genannten Grenzflächen definiert wird und die in der Hornhaut liegenden Grenzflächen mittels Photodisruption erzeugt werden. Der Volumenkörper kann dabei lentikelartig ausgebildet sein.

Zudem kann der Laser derart gesteuert werden, dass das vordefinierte Muster zumindest teilweise kreis- und/oder spiralförmig abgetragen wird. Es besteht auch die Möglichkeit, dass das vordefinierte Muster anhand eines oder mehrerer Steuerdatensätze definiert ist, wobei der oder die Steuerdatensätze Steuerdaten zur Positionierung und/oder zur Fokussierung einzelner Laserpulse in der Hornhaut umfassen. Die Steuerdatensätze werden zumindest durch ein Bereitstellen von topographischen und/oder pachymetrischen und/oder morphologischen Daten der unbehandelten Hornhaut und ein Bereitstellen von topographischen und/oder pachymetrischen und/oder morphologischen Daten des zu entfernenden krankhaft veränderten Bereichs innerhalb der Hornhaut, erzeugt.

Der abzutrennende Volumenkörper kann des Weiteren derart ausgebildet sein, dass durch seine Entfernung zudem eine Korrektur einer Fehlsichtigkeit des Auges erfolgt. Der krankhaft veränderte Bereich innerhalb der Hornhaut kann eine Trübung und/oder eine Narbe sein. Des Weiteren besteht die Möglichkeit, dass die Steuereinrichtung derart ausgebildet ist, dass der Laser Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt.

Weitere Merkmale und deren Vorteile sind den Beschreibungen des ersten Erfindungsaspekts zu entnehmen, wobei vorteilhafte Ausgestaltungen jedes Erfindungsaspekts als vorteilhafte Ausgestaltungen des jeweils anderen Erfindungsaspekts anzusehen sind.

Die Erfindung ist durch die Ansprüche definiert.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Darstellung einer erfindungsgemäßen Behandlungsvorrichtung; und
- Fig. 2: eine Prinzipdarstellung der Erzeugung eines abzutrennenden Volumenkörpers gemäß der ersten Ausführungsform des erfindungsgemäßen Computerprogramms

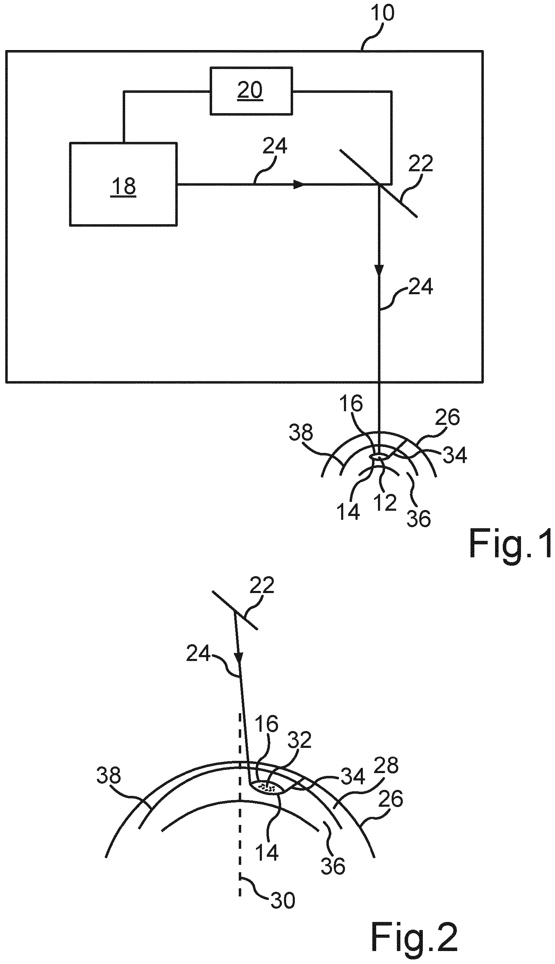

Figur 1 zeigt eine schematische Darstellung einer Behandlungsvorrichtung 10 mit einem augenchirurgischen Laser 18 für die Abtrennung eines vordefinierten Hornhautvolumens beziehungsweise Volumenkörpers 12 mit vordefinierten Grenzflächen 14, 16 einer Hornhaut eines menschlichen oder tierischen Auges mittels Photodisruption. Man erkennt, dass neben dem Laser 18 eine Steuereinrichtung 20 für den Laser 18 ausgebildet ist, sodass dieser gepulste Laserpulse in einem vordefinierten Muster in die Hornhaut abgibt, wobei die Grenzflächen 14, 16 des abzutrennenden Volumenkörpers 12 durch das vordefinierte Muster mittels Photodisruption erzeugt werden. Die Grenzflächen 14, 16 bilden in dem dargestellten Ausführungsbeispiel einen lentikelartigen Volumenkörper 12 aus, wobei die Position des Volumenkörpers 12 derart gewählt ist, dass ein krankhafter und/oder unnatürlich veränderter Bereich 32 (siehe Figur 2) innerhalb der Stroma 36 der Hornhaut umschlossen wird. Des Weiteren ist aus Figur 1 erkennbar, dass zwischen der Stroma 36 und dem Epithelium 28 die so genannten Bowman Membran 38 ausgebildet ist.

Des Weiteren erkennt man, dass der durch den Laser 18 erzeugte Laserstrahl 24 mittels einer Strahleinrichtung 22, nämlich einer Strahlablenkungsvorrichtung, wie zum Beispiel einem Scanner, in Richtung einer Oberfläche 26 der Hornhaut abgelenkt wird. Die Strahlablenkvorrichtung 22 wird ebenfalls durch die Steuereinrichtung 20 gesteuert um das genannte vordefinierte Muster in der Hornhaut zu erzeugen.

Bei dem dargestellten Laser 18 handelt es sich um einen photodisruptiven Laser der ausgebildet ist, Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

Die Steuereinrichtung 20 weist zudem eine Speichereinrichtung (nicht dargestellt) zur zumindest temporären Speicherung von mindestens einem Steuerdatensatz auf, wobei der oder die Steuerdatensätze Steuerdaten zur Positionierung und/oder zur Fokussierung einzelner Laserpulse in der Hornhaut umfassen. Die Positionsdaten und/oder Fokussierungsdaten der einzelnen Laserpulse werden anhand einer zuvor gemessenen Topografie und/oder Pachymetrie und//oder der Morphologie der Hornhaut und dem zu entfernenden, krankhaften und/oder unnatürlich veränderten Bereich 32 innerhalb der Stroma 36 des Auges erzeugt.

Figur 2 zeigt eine Prinzipdarstellung der Erzeugung des abzutrennenden Volumenkörpers 12 gemäß einer Ausführungsform des vorliegenden Computerprogramms. Man erkennt, dass mittels des gepulsten Laserstrahls 24, der über die Strahlablenkvorrichtung 22 in Richtung der Hornhaut beziehungsweise in Richtung der Oberfläche 26 der Hornhaut gelenkt wird, die Grenzflächen 14, 16 erzeugt werden. Die Grenzflächen 14, 16 bilden dabei einen lentikelartigen Volumenkörper aus, der den krankhaften und/oder unnatürlich veränderten Bereich 32 innerhalb der Stroma 36 umschließt. Des Weiteren erzeugt in dem dargestellten Ausführungsbeispiel der Laser 18 einen weiteren Schnitt 34, der in einem vordefinierten Winkel und mit einer vordefinierten Geometrie den Volumenkörper schneidet und bis zu der Oberfläche 26 der Hornhaut ausgebildet ist. Der durch die Grenzflächen 14, 16 definierte Volumenkörper kann dann über den Schnitt 34 aus der Hornhaut entfernt werden. In dem dargestellten Ausführungsbeispiel ist der krankhafte und/oder unnatürlich veränderte Bereich 32 innerhalb der Stroma 36 und außerhalb einer optischen Achse 30 des Auges ausgebildet.

In dem dargestellten Ausführungsbeispiel wird zunächst mittels dem Laserstrahl 24 die Grenzfläche 14, das heißt die tiefer im Auge beziehungsweise der Stroma 36 liegende Grenzfläche ausgebildet. Dies kann durch ein zumindest teilweise kreis- und/oder spiralförmiges Führen des Laserstrahls 24 gemäß dem vordefinierten Muster erfolgen. Anschließend wird auf vergleichbare Art und Weise die Grenzfläche 16 erzeugt, sodass die Grenzflächen 14, 16 den lentikelförmigen Volumenkörper 12 (siehe auch Fig. 1) ausbilden. Anschließend wird der Schnitt 34 ebenfalls mit dem Laser 18 erzeugt. Die Reihenfolge der Erzeugung der Grenzflächen 14, 16 und des Schnitts 34 kann jedoch auch geändert werden.

## Patentansprüche

1. Computerprogramm zur Steuerung eines augenchirurgischen Lasers (18) für die Abtrennung eines Volumenkörpers (12) mit vordefinierten Grenzflächen (14, 16) aus einer menschlichen oder tierischen Hornhaut, umfassend Befehle, die bewirken, dass eine Behandlungsvorrichtung (10) den Laser (18) mittels einer Steuereinrichtung (20) derart steuert, dass dieser gepulste Laserpulse in einem vordefinierten Muster in die Hornhaut abgibt, wobei die Grenzflächen (14, 16) des abzutrennenden Volumenkörpers (12) durch das vordefinierte Muster definiert sind und die Grenzflächen (14, 16) mittels Photodisruption erzeugt werden, wobei die Grenzflächen (14, 16) des Volumenkörpers (12) derart bestimmt werden, dass ein krankhafter und/oder unnatürlich veränderter Bereich (32) innerhalb der Stroma der Hornhaut umschlossen wird,
wobei der Laser (18) derart gesteuert wird, dass mindestens ein Schnitt (34) in einem vordefinierten Winkel und mit einer vordefinierten Geometrie in der Hornhaut erzeugt wird, wobei der Schnitt (34) eine Grenzfläche (14, 16) des Volumenkörpers (12) schneidet und bis zu einer Oberfläche (26) der Hornhaut ausgebildet ist, so dass der Volumenkörper (12) über den Schnitt (34) aus der Hornhaut entfernbar ist und
**dadurch gekennzeichnet, dass** die Oberfläche (26) der Hornhaut eine mittels Abtragen oder Verschieben einer obersten Hornhautschicht und/oder mittels Herstellung einer Hornhautklappe künstlich erzeugte Oberfläche des Auges ist.

2. Computerprogramm nach Anspruch 1, **dadurch gekennzeichnet, dass** der Volumenkörper (12) lentikelartig ausgebildet ist.

3. Computerprogramm nach Anspruch 1 oder 2, **dadurch gekennzeichnet, dass** der Laser (18) derart gesteuert wird, dass das vordefinierte Muster zumindest teilweise kreis- und/oder spiralförmig abgetragen wird.

4. Computerprogramm nach einem der vorhergehenden Ansprüche, **dadurch gekennzeichnet, dass** das vordefinierte Muster anhand eines oder mehrerer Steuerdatensätze definiert ist, wobei der oder die Steuerdatensätze Steuerdaten zur Positionierung und/oder zur Fokussierung einzelner Laserpulse in der Hornhaut umfassen.

5. Computerprogramm nach Anspruch 4, **dadurch gekennzeichnet, dass** die Steuerdatensätze zumindest durch ein Bereitstellen von topographischen und/oder pachymetrischen und/oder morphologischen Daten der unbehandelten Hornhaut und ein Bereitstellen von topographischen und/oder pachymetrischen und/oder morphologischen Daten des zu entfernenden krankhaft veränderten Bereichs (32) innerhalb der Hornhaut, erzeugt werden.

6. Computerprogramm nach einem der vorhergehenden Ansprüche, **dadurch gekennzeichnet, dass** der abzutrennende Volumenkörper (12) des Weiteren derart ausgebildet ist, dass durch die Entfernung des Volumenkörpers (12) zudem eine Korrektur einer Fehlsichtigkeit des Auges erfolgt.

7. Computerprogramm nach einem der vorhergehenden Ansprüche, **dadurch gekennzeichnet, dass** der krankhaft veränderte Bereich (32) innerhalb der Hornhaut eine Trübung und/oder eine Narbe ist.

8. Computerprogramm nach einem der vorhergehenden Ansprüche, **dadurch gekennzeichnet, dass** die Steuereinrichtung (20) derart ausgebildet ist, dass der Laser (18) Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abgibt.

9. Behandlungsvorrichtung (10) mit mindestens einem augenchirurgischen Laser (18) für die Abtrennung eines Hornhautvolumens (12) mit vordefinierten Grenzflächen (14, 16) eines menschlichen oder tierischen Auges mittels Photodisruption und mindestens einer Steuereinrichtung (20) für den oder die Laser (18), die ausgebildet ist, die Schritte des Computerprogramms nach einem der Ansprüche 1 bis 8 auszuführen.

10. Behandlungsvorrichtung nach Anspruch 9, **dadurch gekennzeichnet, dass** der Laser (18) geeignet ist Laserpulse in einem Wellenlängenbereich zwischen 300 nm und 1400 nm, vorzugsweise zwischen 900 nm und 1200 nm, bei einer jeweiligen Pulsdauer zwischen 1 fs und 1 ns, vorzugsweise zwischen 10 fs und 10 ps, und einer Wiederholungsfrequenz größer 10 KHz, vorzugsweise zwischen 100 KHz und 100 MHz, abzugeben.

11. Behandlungsvorrichtung nach Anspruch 9 oder 10, **dadurch gekennzeichnet, dass** die Steuereinrichtung (20)
- mindestens eine Speichereinrichtung zur zumindest temporären Speicherung von mindestens einem Steuerdatensatz aufweist, wobei der oder die Steuerdatensätze Steuerdaten zur Positionierung und/oder zur Fokussierung einzelner Laserpulse in der Hornhaut umfassen; und
- mindestens eine Strahleinrichtung (22) zur Strahlführung und/oder Strahlformung und/oder Strahlablenkung und/oder Strahlfokussierung eines Laserstrahls (24) des Lasers (18) umfasst.

12. Computerlesbares Medium, auf dem das Computerprogramm nach einem der Ansprüche 1 bis 8 gespeichert ist.

## Claims

1. A computer program for controlling an eye surgical laser (18) for the separation of a volume body (12) with predefined interfaces (14, 16) from a human or animal cornea, comprising commands, which cause a treatment device (10) to control the laser (18) by means of a control device (20) such that it emits pulsed laser pulses in a predefined pattern into the cornea, wherein the interfaces (14, 16) of the volume body (12) to be separated are defined by the predefined pattern and the interfaces (14, 16) are generated by means of photodisruption, wherein the interfaces (14, 16) of the volume body (12) are determined such that a pathological and/or unnaturally altered area (32) within the stroma of the cornea is enclosed,
wherein the laser (18) is controlled such that at least one incision (34) is generated in the cornea at a predefined angle and with a predefined geometry, wherein the incision (34) intersects an interface (14, 16) of the volume body (12) and is formed up to a surface (26) of the cornea, such that the volume body (12) is removable from the cornea via the incision (34), and
**characterized in that** the surface (26) of the cornea is a surface of the eye artificially generated by means of ablation or displacement of an uppermost corneal layer and/or by means of production of a corneal flap.

2. The computer program according to claim 1, **characterized in that** the volume body (12) is lenticularly formed.

3. The computer program according to claim 1 or 2, **characterized in that** the laser (18) is controlled such that the predefined pattern is at least partially circularly and/or spirally ablated.

4. The computer program according to any one of the preceding claims, **characterized in that** the predefined pattern is defined based on one or more control datasets, wherein the control dataset or datasets include control data for positioning and/or for focusing individual laser pulses in the cornea.

5. The computer program according to claim 4, **characterized in that** the control datasets are generated at least by providing topographic and/or pachymetric and/or morphologic data of the untreated cornea and providing topographic and/or pachymetric and/or morphologic data of the pathologically altered area (32) to be removed within the cornea.

6. The computer program according to any one of the preceding claims, **characterized in that** the volume body (12) to be separated is furthermore formed such that a correction of a visual disorder of the eye is additionally effected by the removal of the volume body (12).

7. The computer program according to any one of the preceding claims, **characterized in that** the pathologically altered area (32) within the cornea is an opacity and/or a scar.

8. The computer program according to any one of the preceding claims, **characterized in that** the control device (20) is formed such that the laser (18) emits laser pulses in a wavelength range between 300 nm and 1400 nm, preferably between 900 nm and 1200 nm, at a respective pulse duration between 1 fs and 1 ns, preferably between 10 fs and 10 ps, and a repetition frequency of greater than 10 KHz, preferably between 100 KHz and 100 MHz.

9. A treatment device (10) with at least one eye surgical laser (18) for the separation of a corneal volume (12) with predefined interfaces (14, 16) of a human or animal eye by means of photodisruption and at least one control device (20) for the laser or lasers (18), which is formed to execute the steps of the computer program according to any one of claims 1 to 8.

10. The treatment device according to claim 9, **characterized in that** the laser (18) is suitable to emit laser pulses in a wavelength range between 300 nm and 1400 nm, preferably between 900 nm and 1200 nm, at a respective pulse duration between 1 fs and 1 ns, preferably between 10 fs and 10 ps, and a repetition frequency of greater than 10 KHz, preferably between 100 KHz and 100 MHz.

11. The treatment device according to claim 9 or 10, **characterized in that** the control device (20)
- comprises at least one storage device for at least temporary storage of at least one control dataset, wherein the control dataset or datasets include control data for positioning and/or for focusing individual laser pulses in the cornea; and
- comprises at least one beam device (22) for beam guidance and/or beam shaping and/or beam deflection and/or beam focusing of a laser beam (24) of the laser (18).

12. A computer-readable medium, on which the computer program according to any one of claims 1 to 8 is stored.

## Revendications

1. Programme informatique de commande d'un laser chirurgical ophtalmique (18) pour la séparation d'un corps volumique (12) avec des interfaces prédéfinies (14, 16) d'une cornée humaine ou animale, comprenant des instructions qui amènent un dispositif de traitement (10) à commander le laser (18) au moyen d'un dispositif de commande (20), de telle sorte que celui-ci émet des impulsions laser pulsées selon un modèle prédéfini dans la cornée, dans lequel les interfaces (14, 16) du corps volumique (12) à séparer sont définies par le modèle prédéfini et les interfaces (14, 16) sont générées au moyen d'une photodisruption, dans lequel les interfaces (14, 16) du corps volumique (12) sont déterminées de telle sorte qu'une zone pathologique et/ou altérée de manière non naturelle (32) à l'intérieur du stroma de la cornée est entourée,
dans lequel le laser (18) est commandé de telle sorte qu'au moins une incision (34) est générée dans la cornée à un angle prédéfini et avec une géométrie prédéfinie, l'incision (34) coupant une interface (14, 16) du corps volumique (12) et étant formée jusqu'à une surface (26) de la cornée, de sorte que le corps volumique (12) peut être retiré de la cornée via l'incision (34) et
**caractérisé en ce que**
la surface (26) de la cornée est une surface générée artificiellement au moyen d'une ablation et/ou d'un déplacement d'une couche de cornée supérieure et/ou au moyen de la production d'un volet cornéen.

2. Programme informatique selon la revendication 1, **caractérisé en ce que** le corps volumique (12) est formé de manière lenticulaire.

3. Programme informatique selon la revendication 1 ou 2, **caractérisé en ce que** le laser (18) est commandé de telle sorte que le modèle prédéfini est enlevé de manière au moins partiellement circulaire et/ou en spirale.

4. Programme informatique selon l'une des revendications précédentes, **caractérisé en ce que** le modèle prédéfini est défini au moyen d'un ou plusieurs ensembles de données de commande, l'ensemble ou les ensembles de données de commande comprenant des données de commande pour le positionnement et/ou la focalisation d'impulsions laser individuelles dans la cornée.

5. Programme informatique selon la revendication 4, **caractérisé en ce que** les ensembles de données de commande sont générés au moins en fournissant des données topographiques et/ou pachymétriques et/ou morphologiques de la cornée non traitée et en fournissant des données topographiques et/ou pachymétriques et/ou morphologiques de la zone pathologiquement altérée (32) à enlever à l'intérieur de la cornée.

6. Programme informatique selon l'une des revendications précédentes, **caractérisé en ce que** le corps volumique (12) à séparer est en outre formé de telle sorte qu'une correction d'un trouble visuel de l'oeil est de plus réalisée par l'ablation du corps volumique (12).

7. Programme informatique selon l'une des revendications précédentes, **caractérisé en ce que** la zone (32) pathologiquement altérée à l'intérieur de la cornée est une opacité et/ou une cicatrice.

8. Programme informatique selon l'une des revendications précédentes, **caractérisé en ce que** le laser (18) est formé de telle sorte que le laser (18) émet des impulsions laser dans une gamme de longueurs d'onde entre 300 nm et 1 400 nm, de préférence entre 900 nm et 1 200 nm, à une durée d'impulsion respective entre 1 fs et 1 ns, de préférence entre 10 fs et 10 ps, et à une fréquence de répétition supérieure à 10 kHz, de préférence entre 100 kHz et 100 MHz.

9. Dispositif de traitement (10) avec au moins un laser chirurgical ophtalmique (18) pour la séparation d'un volume de cornée (12) avec des interfaces (14, 16) prédéfinies d'un oeil humain ou animal au moyen d'une photodisruption, et au moins un dispositif de commande (20) pour le ou les lasers (18), qui est conçu pour exécuter les étapes du programme informatique selon l'une des revendications 1 à 8.

10. Dispositif de traitement selon la revendication 9, **caractérisé en ce que** le laser (18) est adapté pour émettre des impulsions laser dans une gamme de longueurs d'onde entre 300 nm et 1 400 nm, de préférence entre 900 nm et 1 200 nm, à une durée d'impulsion respective entre 1 fs et 1 ns, de préférence entre 10 fs et 10 ps, et une fréquence de répétition supérieure à 10 kHz, de préférence entre 100 kHz et 100 MHz.

11. Dispositif de traitement selon la revendication 9 ou 10, **caractérisé en ce que** ce dispositif de commande (20) comprend
- au moins un dispositif de stockage pour le stockage au moins temporaire d'au moins un ensemble de données de commande, l'ensemble ou les ensembles de données de commande comprenant des données de commande pour le positionnement et/ou la focalisation d'impulsions laser individuelles dans la cornée ; et
- au moins un dispositif à faisceau (22) pour le guidage de faisceau et/ou la conformation de faisceau et/ou la déviation de faisceau et/ou la focalisation de faisceau d'un faisceau laser (24) du laser (18).

12. Support lisible par ordinateur sur lequel est stocké le programme informatique selon l'une des revendications 1 à 8.
